# DEMANDE DE BREVET EUROPEEN

(11) **EP 4 623 869 A1**
(43) Date de publication de la demande: **01.10.2025**
(21) Numéro de dépôt: 25166363.9
(22) Date de dépôt: 26.03.2025
(51) Int. Cl.: A61F 2/30, A61F 2/40

(54) **ELÉMENT D'ANCRAGE HUMÉRAL DE PROTHÈSE D'ÉPAULE**

(30) Priorité: 27.03.2024 FR 2403065
(71) Demandeur: FX Shoulder Solutions, 01440 Viriat (FR)
(72) Inventeur: ANTONI, Maxime, 68350 Brunstatt (FR); GRESTA, Giorgio, 42170 Saint-Just-Saint-Rambert (FR); HARRIS, Howard, Southlake, 76092 (US); NOURISSAT, Geoffroy, 94300 Vincennes (FR); SRIKUMARAN, Umasuthan, Ellicott City, 12195 (US)
(74) Mandataire: LLR

(57) **Abrégé**

L'invention a pour objet un élément d'ancrage huméral (2) d'une pièce articulaire humérale (4, 4') de prothèse d'épaule, l'élément d'ancrage huméral (2) étant caractérisé en ce qu'il comprend :
- un corps principal (8) destiné à être inséré à l'intérieur d'un humérus (16) à partir d'une surface métaphysaire réséquée (10), et
- une collerette proximale (12) formant une extrémité proximale de l'élément d'ancrage huméral (2) et placée à une extrémité du corps principal (8), une face proximale (14) de la collerette proximale (12) s'étendant dans l'humérus (16) et en-dessous de la surface métaphysaire réséquée (10).

## Description

La présente invention concerne un élément d'ancrage huméral permettant l'ancrage à un humérus d'une pièce articulaire humérale d'une prothèse d'épaule.

Il est bien connu de réaliser l'ancrage d'une pièce articulaire humérale au moyen d'une tige médullaire effilée que comprend cette pièce. La tige médullaire présente une partie proximale évasée, destinée à prendre place au niveau de la métaphyse osseuse, et une partie distale effilée destinée à être insérée dans le canal médullaire jusqu'au niveau de la diaphyse. Une telle pièce articulaire à tige médullaire peut cependant être délicate à implanter lorsque l'os n'est pas de bonne qualité, particulièrement en cas d'ostéoporose sur un patient âgé. Il en est de même en cas de reprise d'une prothèse antérieurement implantée, cette implantation antérieure ayant déjà conduit à une ablation d'os plus ou moins importante. Dans un cas comme dans l'autre, fixation de la pièce articulaire par rapport à l'os peut ne pas être parfaite et ne pas parfaitement résister aux contraintes répétées subies par la prothèse au cours du temps.

Dans un autre cas de figure, et pour un patient comprenant un os sain (patient par exemple plus jeune) mais une articulation usée par de nombreuses sollicitations (par exemple dues au travail ou à la pratique sportive) et/ou pour lequel une intervention au niveau des muscles de la coiffe des rotateurs n'est pas ou plus possible, l'amélioration de la tenue des prothèses dans le temps permet de réaliser une pose de prothèse en utilisant un élément d'ancrage dépourvu de tige médullaire. Cela est dû au fait qu'il est possible de stabiliser la prothèse sans avoir à aller chercher un point d'ancrage assez bas dans la partie diaphysaire de l'humérus car l'épiphyse et la métaphyse sont de bonne qualité. L'élément d'ancrage s'étend alors dans la partie métaphysaire de l'os, après résection de la tête humérale.

Dans le cas d'une prothèse inversée, l'empilement des éléments formant la pièce articulaire humérale peut conduire à un déplacement du centre de rotation de l'articulation de l'humérus vers la glène. Cela n'est pas optimal car, sans prothèse, le centre de rotation de l'articulation anatomique se situe au niveau de l'humérus et non de la glène. La pose d'une prothèse peut dès lors conduire à obtenir un déplacement du centre de rotation de l'articulation pour créer une situation qui ne reproduit pas la situation articulaire anatomique.

La présente invention a notamment pour but de remédier à cet inconvénient en fournissant un élément d'ancrage huméral permettant de conserver un positionnement du centre de rotation de l'articulation le plus proche possible d'une réalité anatomique de l'articulation tout en mettant en œuvre un élément d'ancrage le moins invasif possible.

A cet effet, l'invention a pour objet un élément d'ancrage huméral d'une pièce articulaire humérale de prothèse d'épaule, l'élément d'ancrage huméral comprenant :
- un corps principal destiné à être inséré à l'intérieur d'un humérus à partir d'une surface métaphysaire réséquée, et
- une collerette proximale formant une extrémité proximale de l'élément d'ancrage huméral et placée à une extrémité du corps principal, une face proximale de la collerette proximale s'étendant dans l'humérus et en-dessous de la surface métaphysaire réséquée.

Ainsi, on place l'élément d'ancrage intégralement à l'intérieur de l'humérus et en-dessous de la surface métaphysaire réséquée. Cela permet de déplacer l'ensemble de la pièce articulaire humérale vers l'humérus en cas de prothèse d'épaule inversée (élément d'ancrage destiné à recevoir une cupule). Ce déplacement de l'articulation en direction de l'humérus permet, lorsque cela n'est pas le cas pour une prothèse selon l'art antérieur, un déplacement du centre de rotation de l'articulation en direction de l'humérus de sorte à reproduire une situation anatomique dans laquelle le centre de rotation de l'articulation se situe au niveau de l'humérus et non au niveau de la glène. Dans tous les cas de prothèse (anatomique ou inversée), on obtient un élément d'ancrage le moins invasif possible.

Suivant d'autres caractéristiques optionnelles de l'élément d'ancrage huméral prises seules ou en combinaison :
- La collerette proximale est destinée à s'étendre sensiblement parallèlement à un plan dans lequel s'étend la surface métaphysaire réséquée,
- la face proximale de la collerette proximale est destinée à s'étendre dans l'humérus à une distance comprise entre 1 et 10 millimètres en-dessous de la surface métaphysaire réséquée, de préférence entre 1 et 5 millimètres en-dessous de la surface métaphysaire réséquée,
- le corps principal est destiné à s'étendre uniquement dans une partie métaphysaire de l'humérus, le corps principal comprenant un système de rétention périphérique formé par plusieurs plaques d'ancrage périphériques séparées les unes des autres et destinées à s'étendre à partir de la collerette proximale vers une partie distale de l'humérus, deux plaques d'ancrage périphériques adjacentes s'étendant dans des plans non parallèles entre eux,

- les plaques d'ancrage périphériques s'étendent dans une direction sensiblement perpendiculaire à un plan dans lequel s'étend la face proximale de la collerette proximale,
- les plaques d'ancrage périphériques sont de largeur irrégulière le long de leur direction d'extension,
- le corps principal comprend au moins un plot de stabilisation destiné à s'étendre à partir de la collerette proximale vers une partie distale de l'humérus,
- le plot de stabilisation s'étend dans une direction sensiblement perpendiculaire à un plan dans lequel s'étend la face proximale de la collerette proximale,
- le plot de stabilisation comprend des stries de rétention s'étendant sensiblement parallèlement à un axe longitudinal du plot de stabilisation,
- le plot de stabilisation est un corps creux ouvert à son extrémité distale et comprend une pluralité d'orifices traversant ménagés dans sa paroi,
- le plot de stabilisation est un corps creux de forme tubulaire ouvert à son extrémité proximale et comprend un taraudage ménagé sur une surface interne du plot de stabilisation, et
- le plot de stabilisation comprend au moins une couronne latérale s'étendant depuis une face externe du plot de stabilisation dans une direction sensiblement perpendiculaire par rapport à un axe longitudinal du plot de stabilisation.

L'invention a également pour objet une pièce articulaire huméral de prothèse d'épaule comprenant un élément d'ancrage huméral selon l'invention.

La pièce articulaire humérale peut comprendre une tête humérale de remplacement, et avantageusement une entretoise placée entre l'élément d'ancrage huméral et la tête humérale de remplacement.

Alternativement, la pièce articulaire humérale peut comprendre une cupule de réception d'une glénosphère.

La pièce articulaire humérale peut comprendre une partie diaphysaire, la partie diaphysaire étant typiquement formée par une tige humérale.

La pièce articulaire humérale peut en outre comprendre un cône morse mâle rapporté sur la partie diaphysaire et un cône morse femelle porté par l'élément d'ancrage huméral.

L'invention a également pour objet un kit d'implantation d'une pièce articulaire humérale selon l'invention, le kit comprenant plusieurs éléments d'ancrage huméraux selon l'invention de différents diamètres et plusieurs parties diaphysaires de différentes longueurs.

Le kit d'implantation peut comprendre au moins une vis de fixation d'un élément d'ancrage huméral à une partie diaphysaire.

Le kit d'implantation peut comprendre au moins un cône morse mâle destiné à être rapporté sur la partie diaphysaire.

Avantageusement, le kit d'implantation peut comprendre au moins une entretoise et au moins une tête humérale de remplacement et/ou au moins une cupule.

L'invention a également pour objet une prothèse d'épaule comprenant une pièce articulaire humérale selon l'invention.

La prothèse d'épaule peut être une prothèse anatomique ou une prothèse inversée.

### Brève description des figures

L'invention sera mieux comprise à la lecture de la description qui va suivre donnée uniquement à titre d'exemple et faite en se référant aux dessins annexés dans lesquels :
[Fig. 1] est une première vue en perspective d'un élément d'ancrage huméral selon l'invention,
[Fig. 2] est une seconde vue en perspective d'un élément d'ancrage huméral selon l'invention,
[Fig. 3] est une vue de côté d'un élément d'ancrage huméral selon l'invention,
[Fig. 4] est une vue en coupe longitudinale d'un élément d'ancrage huméral selon l'invention,
[Fig. 5] est une vue éclatée et en perspective d'une pièce articulaire humérale selon une première variante du premier mode de réalisation de l'invention,
[Fig. 6] est une vue assemblée et de côté de la pièce articulaire humérale selon la première variante du premier mode de réalisation de l'invention,
[Fig. 7] est une vue éclatée et en perspective d'une pièce articulaire humérale selon une deuxième variante du premier mode de réalisation de l'invention,
[Fig. 8] est une vue éclatée et en perspective d'une prothèse d'épaule inversée comprenant une pièce articulaire humérale selon la deuxième variante du premier mode de réalisation de l'invention,
[Fig. 9] est une vue assemblée et de côté d'une prothèse d'épaule inversée comprenant une pièce articulaire humérale selon la deuxième variante du premier mode de réalisation de l'invention,
[Fig. 10] est une vue de côté d'une pièce articulaire humérale selon la deuxième variante du premier mode de réalisation de l'invention implanté dans un humérus,
[Fig. 11] est une vue de côté d'un ensemble de pièces articulaires humérales selon un deuxième mode de réalisation de l'invention, et
[Fig. 12] est un algorithme global d'une prothèse selon le deuxième mode de réalisation de l'invention, la prothèse étant une prothèse anatomique ou inversée.

### Description détaillée

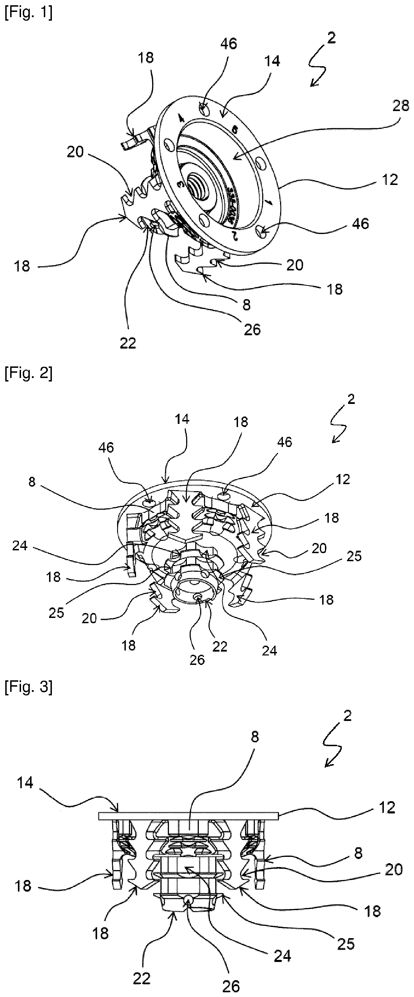

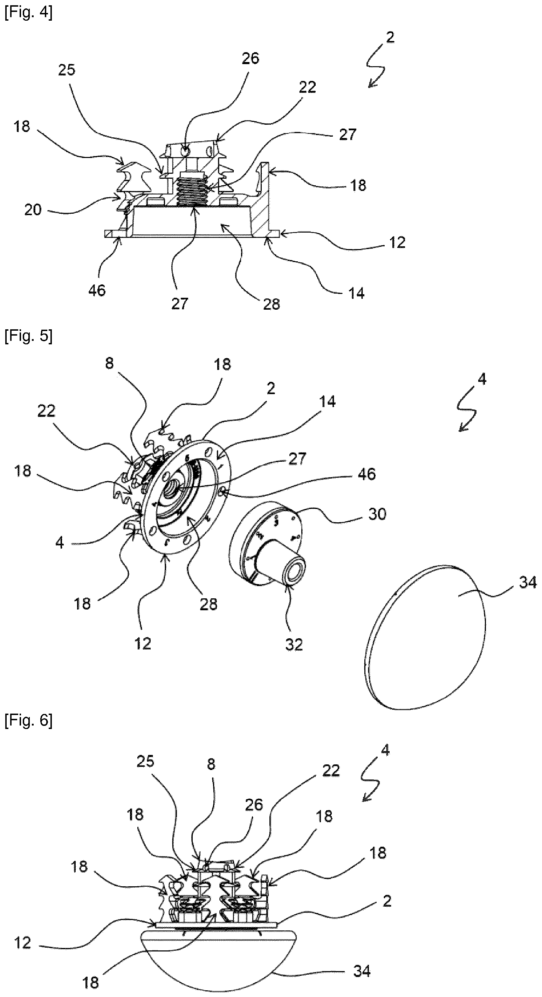

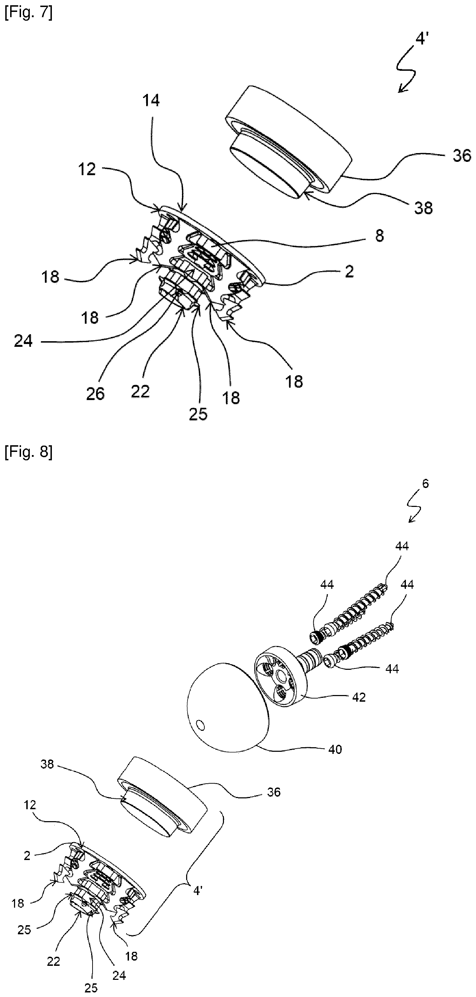

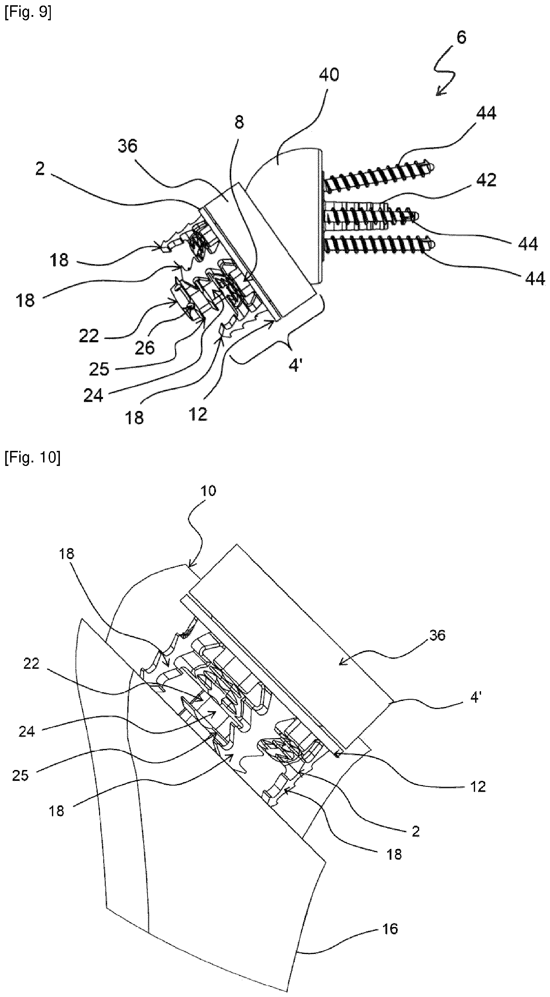

On se réfère désormais aux figures 1 à 4 illustrant un élément d'ancrage huméral 2 d'une pièce articulaire humérale 4 ou 4' (voir figures 5 à 9) de prothèse d'épaule (voir figures 8 et 9 : vues éclatée et assemblée d'une prothèse d'épaule inversée). On entend par élément d'ancrage huméral une partie d'une pièce articulaire humérale destinée à être implanté dans **une cavité** ménagée dans une extrémité proximale d'un humérus, que la pièce articulaire humérale soit celui d'une prothèse d'épaule anatomique ou inversée. L'élément d'ancrage huméral 2 peut être réalisé à partir d'un métal biocompatible tel qu'un alliage de titane ou de l'inox.

L'élément d'ancrage huméral 2 comprend un corps principal 8 destiné à être inséré à l'intérieur de l'humérus à partir d'une surface métaphysaire réséquée 10 (voir figure 10). Il comprend également une collerette proximale 12 placée à une extrémité proximale du corps principal 8 et pouvant être destinée à s'étendre sensiblement parallèlement à la surface métaphysaire réséquée 10.

Une face proximale 14 de la collerette proximale 12 s'étend dans l'humérus et en-dessous de la surface métaphysaire réséquée 10. On entend par cela que l'extrémité proximale de l'élément d'ancrage huméral 2, destinée à être placé au plus près de l'extrémité proximale de l'humérus, est située à l'intérieur de l'humérus et sous le plan formé par la surface métaphysaire réséquée 10. Cela permet, comme expliqué ci-dessus, « d'enfouir » l'élément d'ancrage huméral 2 dans l'humérus et donc de déplacer la pièce articulaire humérale et par conséquent la prothèse d'épaule vers l'humérus, permettant ainsi de placer le centre de rotation de l'articulation au plus près de l'humérus.

La face proximale 14 de la collerette proximale 12 peut par exemple s'étendre dans l'humérus à une distance comprise entre 1 et 10 millimètres en-dessous de la surface métaphysaire réséquée 10, de préférence entre 1 et 5 millimètres en-dessous de la surface métaphysaire réséquée.

La collerette proximale 12 est d'un diamètre compris entre 30 et 48 millimètres.

Avantageusement, et selon un premier mode de réalisation de l'invention, le corps principal 8 s'étend uniquement dans une partie métaphysaire de l'humérus 16. Cela signifie que l'élément d'ancrage 2 est dépourvu de tige médullaire d'ancrage. Cela est particulièrement avantageux, comme expliqué ci-dessus, pour des patients ayant une partie métaphysaire de l'humérus saine et pouvant servir de base d'ancrage pour l'élément d'ancrage huméral 2 qui va notamment pouvoir être placé en appui contre le calcar de la tête humérale qui est avantageusement conservé au moment de la résection osseuse. Une autre possibilité d'implantation d'un élément d'ancrage selon le premier mode de réalisation de l'invention correspond à une implantation pour un patient dont la qualité osseuse n'est pas optimale, mais présentant une déformation de l'humérus empêchant la pose d'une partie diaphysaire, typiquement d'une tige humérale.

Le corps principal 8 peut comprendre un système d'ancrage périphérique formé par plusieurs plaques d'ancrage périphériques 18 séparés les unes des autres, cinq dans l'exemple représenté sur les figures, destinés à s'étendre à partir de la collerette proximale 12 vers une partie distale de l'humérus 16. Dans l'exemple illustré, ces derniers s'étendent à partir de la collerette proximale 12 sensiblement perpendiculairement par rapport à un plan dans lequel s'étend la face proximale 14 de la collerette proximale 12, deux plaques d'ancrage périphériques 18 adjacentes s'étendant dans des plans non parallèles entre eux. L'avantage d'une utilisation d'un ancrage périphérique est de réaliser ledit ancrage dans la partie corticale de l'humérus qui est la partie la plus dense de l'os. Ainsi, on optimise l'ancrage de l'élément d'ancrage huméral 2 dans l'humérus 16 par interaction avec la partie la plus dure de l'humérus 16. La mise en place de plaques d'ancrage périphériques 18 avec des plaques adjacentes ne s'étendant pas dans des plans parallèles entre eux permet d'empêcher une rotation de l'élément d'ancrage 2 dans l'humérus 16.

Avantageusement, les plaques d'ancrage périphériques 18 peuvent être de largeur irrégulière le long de leur direction d'extension. On observe cela sur les figures sur lesquelles des évidements 20 sont présents de part et d'autre des plaques d'extension périphériques 18 sur les côtés de ces dernières. Ces irrégularités permettent d'assurer un bon ancrage de l'élément d'ancrage huméral 2 dans l'os.

De préférence, une ou des portions des côtés des plaques d'ancrage périphériques s'étendent dans une direction parallèle à une direction d'extension de la face proximale 14 de la collerette proximale 12. Cela est visible sur les figures sur lesquelles chaque côté de chaque plaque d'ancrage périphérique 18 comprend trois portions s'étendant dans une telle direction. Cela favorise une fois de plus l'ancrage de l'élément d'ancrage 2 dans l'humérus 16.

La longueur des plaques d'ancrage périphériques 18 est avantageusement comprise entre 12 et 20 millimètres.

Le corps principal 8 peut comprendre un plot de stabilisation 22, placé en position centrale dans l'exemple des figures, destiné à s'étendre à partir de la collerette proximale 12 vers une partie distale de l'humérus 16. Dans l'exemple illustré, ce dernier s'étend à partir de la collerette proximale 12 sensiblement perpendiculairement par rapport à un plan dans lequel s'étend la face proximale 14 de la collerette proximale 12. Ce plot de stabilisation 22 a pour but d'éviter un renversement de l'élément d'ancrage 2 et donc de la pièce articulaire 4 ou 4' dans l'humérus 16.

La longueur du plot de stabilisation est avantageusement comprise entre 14 et 30 millimètres.

Le plot de stabilisation 22 peut avantageusement comprendre des stries de rétention 24 s'étendant sensiblement parallèlement à un axe longitudinal du plot de stabilisation 22. Ces dernières permettent d'optimiser la fonction anti renversement du plot de stabilisation 22.

Le plot de stabilisation 22 peut en outre être un corps creux ouvert à son extrémité distale (potentiellement à son extrémité proximale et à son extrémité distale) et comprendre des orifices traversant 26 ménagés dans sa paroi. Ces derniers permettent de relier l'intérieur du plot de stabilisation 22 à l'extérieur de ce dernier. Cela permet une colonisation osseuse et une vascularisation de la portion d'os se situant à l'intérieur du plot de stabilisation 22. Cette vascularisation permet de conserver un os consistant à l'intérieur du plot de stabilisation 22, ce qui optimise la stabilisation de l'élément d'ancrage huméral 2 dans l'humérus 16 en permettant un ancrage par l'intérieur du plot de stabilisation 22.

Le plot de stabilisation 22 peut en outre être un corps creux ouvert à son extrémité proximale (potentiellement à son extrémité proximale et à son extrémité distale) et comprendre un taraudage 27 ménagé sur une surface interne du plot de stabilisation 22. Ce taraudage 27 permet l'ablation de l'élément d'ancrage huméral 2 de l'humérus 16 si cela est nécessaire et par vissage d'un outil d'extraction de l'élément d'ancrage huméral 2. Il serait possible d'utiliser un système de fixation autre qu'un taraudage, comme un système de crans ou d'encoches.

Le plot de stabilisation peut en outre comprendre au moins une couronne latérale 25 (deux sur les figures) s'étendant depuis une face externe du plot de stabilisation 22 dans une direction sensiblement perpendiculaire par rapport à un axe longitudinal du plot de stabilisation 22. Elle permet également d'optimiser l'ancrage de l'élément d'ancrage huméral 2 dans l'os. La couronne latérale 25 peut en outre comprendre des encoches alignées avec les stries de rétention 24 afin de conserver la fonction de ces dernières. Les stries de rétention ne sont de manière générale pas continues et ce afin d'avoir de l'os se positionnant entre les portions de stries de rétention 24 afin d'éviter un peu plus la rotation de l'élément d'ancrage huméral 2.

L'élément d'ancrage huméral 2 peut en outre comprendre un renfoncement 28 pouvant former un système de fixation conique destiné à recevoir une autre partie d'une pièce articulaire humérale 4 ou 4' (cupule ou entretoise) comme cela va être décrit par la suite.

La collerette proximale 12 peut en outre comprendre des trous de suture 46 (illustrés sur les figures 1 à 4) permettant de rattacher les parties molles de l'os à l'élément d'ancrage huméral 2.

La figure 5 illustre une pièce articulaire humérale 4 selon un premier mode de réalisation de l'invention et dans le cas où la prothèse d'épaule est une prothèse anatomique, c'est-à-dire reproduisant la forme de l'articulation anatomique. La pièce articulaire humérale 4 comprend, en plus de l'élément d'ancrage huméral 2, une entretoise 30 destinée à être disposée dans le renfoncement 28. L'entretoise 30 comprend un plot de fixation 32 destiné à recevoir une tête humérale de remplacement 34.

La figure 6 illustre quant à elle une pièce articulaire humérale 4 selon une première variante du premier mode de réalisation de l'invention et dans le cas où la prothèse d'épaule est une prothèse anatomique, la pièce articulaire humérale 4 étant assemblé. L'entretoise 30 est placée dans le renfoncement 28 et le plot de réception 32 est inséré dans un orifice de fixation ménagé dans la tête humérale de remplacement 34.

La figure 7 illustre une pièce articulaire humérale 4' selon une deuxième variante du premier mode de réalisation de l'invention. Dans ce mode de réalisation, la pièce articulaire humérale 4' est celui d'une prothèse d'épaule inversée 6 visible sur la figure 8. Une cupule 36 est destinée à être montée sur l'élément d'ancrage huméral 2. Cette cupule 36 comprend, au niveau de son extrémité placée face à la glène, une surface concave destinée à recevoir une glénosphère 40 (visible sur les figures 8 et 9). Des cupules de différentes hauteurs peuvent être utilisés avec le même élément d'ancrage huméral 2.

Dans ce mode de réalisation, une embase 38 de la cupule 36 est destinée à être placé dans le renfoncement 27. L'embase 38 peut être de forme conique et être assez large, avec par exemple un diamètre au plan de jauge compris entre 15 et 30 millimètres.

La cupule 36 est avantageusement la plus large possible afin d'y ménager une surface concave la plus profonde possible. Cela permet d'avoir une couche de polyéthylène dans la surface concave, d'une épaisseur par exemple égale à 4 millimètres, contre laquelle la glénosphère 40 est placée tout en permettant à la glénosphère 40 d'être placée le plus profondément possible dans la surface concave.

Les figures 8 et 9 illustrent une prothèse d'épaule inversée 6, respectivement en vue éclatée (figure 8) et en vue assemblée (figure 9). Il comprend la pièce articulaire humérale 4', et une pièce articulaire glénoïdienne comprenant dans l'exemple illustré la glénosphère 40, une métaglène 42 destinée à être fixée dans la glène et sur laquelle est fixée la glénosphère 40. Plusieurs vis d'ancrage 44 sont destinées à être utilisées pour fixer la métaglène 42 dans la glène.

La figure 10 illustre, comme décrit ci-dessus, une portion proximale d'un humérus 16 présentant une surface métaphysaire réséquée 10 et une pièce articulaire humérale 4' selon la deuxième variante du premier mode de réalisation de l'invention. Comme cela est visible sur la figure, l'intégralité de l'élément d'ancrage 2 est disposé à l'intérieur de l'humérus 16, la face proximale 14 de la collerette proximale 12 s'étend en-dessous d'un plan dans lequel s'étend la surface métaphysaire réséquée 10.

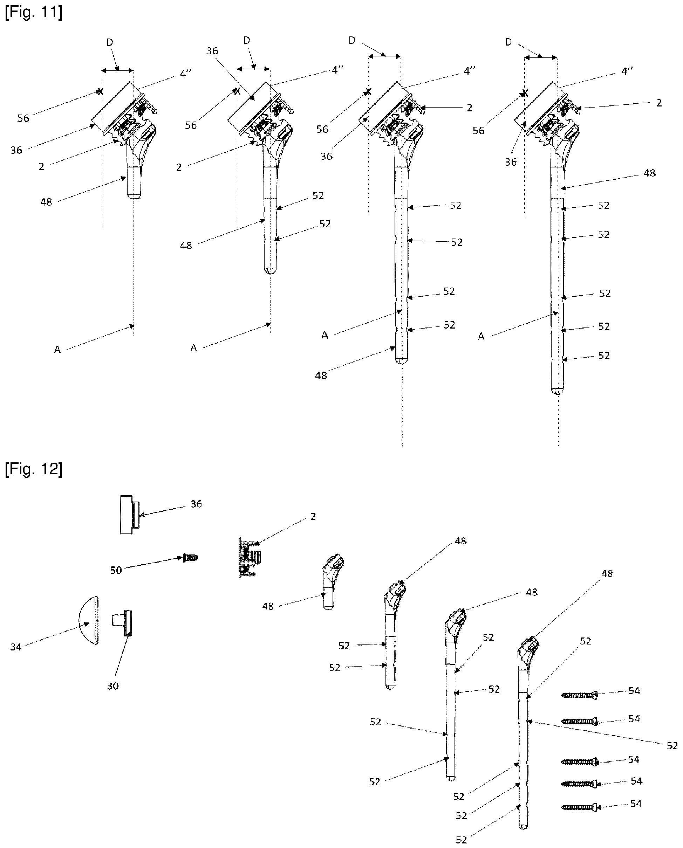

Les figures 11 et 12 illustrent quant à elles un deuxième mode de réalisation de l'invention d'une pièce articulaire humérale 4". Dans ce dernier, la pièce articulaire humérale comprend un élément d'ancrage huméral 2 coopérant avec une cupule 36 afin de former une pièce articulaire humérale 4'. Bien entendu, il est possible de placer sur l'élément d'ancrage huméral 2 une tête humérale de remplacement 34 avec une entretoise 30 afin de former une pièce articulaire humérale 4.

La pièce articulaire humérale 4 ou 4' peut être fixée sur une partie diaphysaire, par exemple une tige humérale 48, par exemple par l'intermédiaire d'une vis de fixation 50. La partie diaphysaire peut être de différente longueur comme cela est illustré sur les figures 11 et 12 avec des tiges humérales de différentes longueurs et ce en fonction du patient devant recevoir une pièce articulaire humérale 4" selon le deuxième mode de réalisation de l'invention.

L'utilisation d'une partie diaphysaire est particulièrement indiquée dans le cadre d'un patient dont la partie métaphysaire de l'humérus est détériorée. Dans un tel cas de figure, une fixation d'une pièce articulaire humérale 4 ou 4' selon le premier mode de réalisation de l'invention est difficilement envisageable car cela pourrait conduire à un défaut de stabilité de la pièce articulaire humérale 4 ou 4'. Dans un but de stabilisation, la partie diaphysaire est implantée dans l'os, par exemple par extension d'une tige humérale 48 dans la partie médullaire de l'humérus. Un cône morse mâle est ensuite placé sur la partie diaphysaire. L'élément d'ancrage huméral 2 est ensuite monté sur la partie diaphysaire (par une interaction cône morse mâle (monté sur la partie diaphysaire)/cône morse femelle (porté par l'élément d'ancrage huméral 2)) et fixé à cette dernière par l'intermédiaire de la vis de fixation 50. Une fois que l'élément d'ancrage huméral 2 est mis en place et fixé, une cupule 36 ou un ensemble entretoise 30/tête humérale de remplacement 34 peut être rapporté sur l'élément d'ancrage huméral 2 afin de former la pièce articulaire humérale 4". Il faut noter que la partie diaphysaire pourrait comprendre un cône mâle intégré dans la partie diaphysaire et pouvant interagir avec le cône morse femelle porté par l'élément d'ancrage huméral 2.

En fonction de sa longueur, la partie diaphysaire, par exemple la tige humérale 48, peut être pourvue d'orifices de verrouillage 52 coopérant avec des vis de verrouillage 54 afin de verrouiller la partie diaphysaire, et donc la pièce articulaire humérale 4", dans l'humérus.

Comme illustré sur la figure 11, la partie diaphysaire peut être de longueur différente, avec ou sans vis de verrouillage 54 et ce en fonction de l'os du patient dans lequel cette dernière est implantée. Les vis de verrouillage 54 permettent de stabiliser la partie diaphysaire lorsque celle-ci atteint une certaine longueur.

L'invention a également pour objet un kit d'implantation d'une pièce articulaire humérale 4" selon l'invention, le kit comprenant plusieurs éléments d'ancrage huméraux 2 de différents diamètres et plusieurs parties diaphysaires, par exemple plusieurs tige humérales 48, de différentes longueurs. Bien entendu, le kit d'implantation peut également comprendre plusieurs cupules 36 de tailles différentes, plusieurs têtes humérales de remplacement 34 et plusieurs entretoises 30 de tailles différentes et/ou plusieurs vis de fixation 50 de tailles différentes.

Chaque élément d'ancrage huméral 2 du kit d'implantation peut être monté et fixé sur chaque partie diaphysaire du kit d'implantation. Le choix d'une association d'un élément d'ancrage huméral 2 particulier avec une partie diaphysaire particulière dépend du patient, tout comme le choix d'utiliser une tête humérale de remplacement 34 ou une cupule 36 en fonction du type de prothèse d'épaule (anatomique ou inversée). Il est possible, en fonction du diamètre de l'élément d'ancrage huméral 2, de modifier la profondeur d'implantation de la partie diaphysaire de manière à avoir un élément d'ancrage huméral 2 placé à la même hauteur quelle que soit la partie diaphysaire utilisée. En effet, plus le diamètre de l'élément d'ancrage huméral 2 est important et plus la longueur du plot de stabilisation 22 est importante. Il est donc nécessaire d'implanter la partie diaphysaire plus profondément qu'avec un élément d'ancrage huméral 2 de plus petit diamètre, et donc avec un plot de stabilisation 22 plus court, pour que l'élément d'ancrage huméral 2 soit placé à la même hauteur au niveau de la partie métaphysaire de l'humérus.

En ce qui concerne le procédé d'implantation d'une pièce articulaire humérale, deux cas de figures sont à distinguer :
- Pour l'implantation d'une pièce articulaire humérale 4 ou 4' selon le premier mode de réalisation, l'humérus est tout d'abord préparé pour l'implantation : la tête humérale est réséquée et la partie métaphysaire est préparée pour recevoir l'élément d'ancrage huméral 2. Ce dernier est impacté dans la partie métaphysaire de l'humérus et est stable dans cette dernière grâce aux plaques d'ancrage périphériques 18 s'étendant dans la partie métaphysaire de l'humérus (une stabilité supplémentaire est obtenue lors de la cicatrisation par des éléments supplémentaires décrits ci-dessus comme par exemple les orifices traversants 26). Un ensemble entretoise 30/tête humérale de remplacement 34 ou une cupule 36 est ensuite monté sur l'élément d'ancrage huméral 2 afin d'obtenir la pièce articulaire humérale 4 ou 4' coopérant respectivement avec un implant glénoïdien sous forme de cupule (pour une pièce articulaire humérale 4) ou avec un implant glénoïdien comprenant une glénosphère (pour une pièce articulaire humérale 4'). Comme expliqué ci-dessus, l'intérêt principal du premier mode de réalisation demeure dans la possibilité de poser une prothèse le plus rapidement possible et de la manière la moins invasive possible.
- Pour l'implantation d'une pièce articulaire humérale 4" selon le deuxième mode de réalisation, l'humérus est également préparé pour l'implantation : la tête humérale est réséquée et la partie métaphysaire est préparée pour recevoir l'élément d'ancrage huméral 2. En complément, la partie diaphysaire de l'humérus est préparée par la réalisation d'une cavité humérale destinée à recevoir la partie diaphysaire de la pièce articulaire humérale 4". Le chirurgien choisit pour cela un ensemble éléments d'ancrage huméral 2/partie diaphysaire adapté au patient. Il choisit également le cône morse mâle à rapporter sur la partie diaphysaire et permettant une interaction avec le cône morse femelle porté par l'élément d'ancrage huméral 2. En fonction du diamètre de l'élément d'ancrage huméral 2 et donc de la longueur du plot de stabilisation 22, la profondeur d'implantation de la partie diaphysaire varie, ce qui impacte la taille et notamment la profondeur de la cavité humérale réalisée, et cela dans le but d'obtenir un élément d'ancrage huméral 2 toujours placé à la même hauteur au niveau de la partie métaphysaire de l'humérus et ce quel que soit son diamètre. En particulier, plus le diamètre de l'élément d'ancrage huméral 2 est important et plus la partie diaphysaire est implantée profondément dans l'humérus. Le but ici est de conserver un « offset » ; qui correspond à la distance D entre un axe A représentant la direction d'élongation de la partie diaphysaire et le centre de rotation 56 de l'articulation, inchangé quelle que soit la taille de l'élément d'ancrage huméral 2, et donc du plot de stabilisation 22. Une fois la partie diaphysaire mise en place dans l'humérus, potentiellement avec mise en place de vis de verrouillage 54, le cône morse mâle puis l'élément d'ancrage huméral 2 (portant le cône morse femelle) sont rapportés sur la partie diaphysaire et fixés par l'utilisation d'une vis de fixation 50. Enfin, un ensemble entretoise 30/tête humérale de remplacement 34 ou une cupule 36 est ensuite monté sur l'élément d'ancrage huméral 2 afin d'obtenir la pièce articulaire humérale 4 ou 4' coopérant respectivement avec un implant glénoïdien sous forme de cupule (pour une pièce articulaire humérale 4) ou avec un implant glénoïdien comprenant une glénosphère (pour une pièce articulaire humérale 4').

L'avantage de l'existence des deux modes de réalisation de l'invention est également de permettre à un chirurgien de s'adapter à chaque cas. En effet, étant donné qu'il n'est pas possible de déterminer à l'avance la qualité de l'os d'un patient. Dès lors, l'existence d'un kit comprenant en même temps l'élément d'ancrage huméral 2 pouvant être implanté seul mais également avec une partie diaphysaire permet au chirurgien de s'adapter avec un seul produit à tous les cas de figure. Si l'os du patient est sain ou en cas de déformation de l'humérus et empêchant la pose d'une partie diaphysaire, la pose d'un élément d'ancrage huméral 2 seule est réalisée. Si au contraire l'os du patient est de moindre qualité mais dans lequel une partie diaphysaire peut être implantée, alors un ensemble partie diaphysaire/élément d'ancrage huméral 2 peut être placé. Par exemple, si un chirurgien prévoit de poser un élément d'ancrage huméral 2 seul mais constate que cela n'est pas possible à la vue de l'os du patient, alors il peut s'adapter en ajoutant une partie diaphysaire (et un cône morse mâle ainsi qu'une vis de fixation 50) pour basculer sur le deuxième mode de réalisation de l'invention et ce en gardant le même kit (i.e. sans avoir à basculer vers une autre prothèse, par exemple de design différent).

### Liste de références

2 : élément d'ancrage huméral
4, 4', 4" : pièce articulaire humérale
6 : prothèse d'épaule inversée
8 : corps principal
10 : surface métaphysaire réséquée
12 : collerette proximale
14 : face proximale
16 : humérus
18 : plaques d'ancrage périphériques
20 : évidements
22 : plot de stabilisation
24 : stries de rétention
25 : couronnes latérales
26 : orifices traversants
27 : taraudage
28 : renfoncement
30 : entretoise
32 : plot de fixation
34 : tête humérale de remplacement
36 : cupule
38 : embase
40 : glénosphère
42 : métaglène
44 : vis d'ancrage
46 : trous de suture
48 : tige humérale
50 : vis de fixation
52 : orifices de verrouillage
54 : vis de verrouillage
56 : centre de rotation
A : direction d'extension de la partie diaphysaire
D : distance entre l'axe représentant la direction d'élongation de la partie diaphysaire et le centre de rotation de l'articulation

## Revendications

1. Elément d'ancrage huméral (2) d'une pièce articulaire humérale (4, 4', 4") de prothèse d'épaule, l'élément d'ancrage huméral (2) étant **caractérisé en ce qu'**il comprend :
- un corps principal (8) destiné à être inséré à l'intérieur d'un humérus (16) à partir d'une surface métaphysaire réséquée (10), et
- une collerette proximale (12) formant une extrémité proximale de l'élément d'ancrage huméral (2) et placée à une extrémité du corps principal (8), une face proximale (14) de la collerette proximale (12) s'étendant dans l'humérus (16) et en-dessous de la surface métaphysaire réséquée (10).

2. Elément d'ancrage huméral (2) selon la revendication 1, dans lequel le corps principal (8) est destiné à s'étendre uniquement dans une partie métaphysaire de l'humérus (16), le corps principal (8) comprenant un système d'ancrage périphérique formé par plusieurs plaques d'ancrage périphériques (18) séparées les unes des autres et destinées à s'étendre à partir de la collerette proximale (12) vers une partie distale de l'humérus (16), deux plaques d'ancrage périphériques (18) adjacentes s'étendant dans des plans non parallèles entre eux.

3. Elément d'ancrage huméral (2) selon la revendication 2, dans lequel les plaques d'ancrage périphériques (18) sont de largeur irrégulière le long de leur direction d'extension.

4. Elément d'ancrage huméral (2) selon l'une quelconque des revendications précédentes, dans lequel le corps principal comprend au moins un plot de stabilisation (22) destiné à s'étendre à partir de la collerette proximale (12) vers une partie distale de l'humérus (16).

5. Elément d'ancrage huméral (2) selon la revendication 4, dans lequel le plot de stabilisation (22) comprend des stries de rétention (24) s'étendant sensiblement parallèlement à un axe longitudinal du plot de stabilisation (22).

6. Elément d'ancrage huméral (2) selon l'une quelconque des revendications 4 ou 5, dans lequel le plot de stabilisation (22) est un corps creux de forme tubulaire ouvert à son extrémité proximale et comprend un taraudage (27) ménagé sur une surface interne du plot de stabilisation (22).

7. Elément d'ancrage huméral (2) selon l'une quelconque des revendications 4 à 6, dans lequel le plot de stabilisation (22) comprend au moins une couronne latérale (25) s'étendant depuis une face externe du plot de stabilisation (22) dans une direction sensiblement perpendiculaire par rapport à un axe longitudinal du plot de stabilisation (22).

8. Pièce articulaire humérale (4, 4', 4") comprenant un élément d'ancrage huméral (2) selon l'une quelconque des revendications précédentes.

9. Pièce articulaire humérale (4") selon la revendication 8, comprenant en outre une partie diaphysaire sur laquelle l'élément d'ancrage huméral (2) est fixé.

10. Pièce articulaire humérale (4") selon la revendication 9, dans laquelle la partie diaphysaire est formée par une tige humérale (48).

11. Prothèse d'épaule comprenant une pièce articulaire humérale selon l'une quelconque des revendications 8 à 10.

12. Kit d'implantation d'une pièce articulaire humérale (4") selon l'une quelconque des revendications 8 à 11, comprenant plusieurs éléments d'ancrage huméraux (2) selon l'une quelconque des revendications 1 à 8 de différents diamètres et plusieurs parties diaphysaires de différentes longueurs.

13. Kit d'implantation selon la revendication 12, comprenant au moins une vis de fixation (50) d'une partie diaphysaire à un élément d'ancrage huméral (2).

14. Kit d'implantation selon l'une quelconque des revendications 12 ou 13, comprenant au moins une entretoise (30), au moins une tête humérale de remplacement (34) et/ou au moins une cupule (36).

15. Kit d'implantation selon l'une quelconque des revendications 12 à 14, comprenant des vis de verrouillage de la partie diaphysaire dans un humérus dans lequel la partie diaphysaire est implantée.
